# EUROPEAN PATENT SPECIFICATION

(11) **EP 1 448 241 B1**
(45) Date of publication and mention of the grant of the patent: **11.07.2007**
(21) Application number: 02753508.7
(22) Date of filing: 22.08.2002
(51) Int. Cl.: A61L 15/44, A61F 13/14

(54) **BREAST PAD ASSEMBLY CONTAINING A SKIN BENEFIT INGREDIENT**
BRUSTEINLAGE ENTHALTEND HAUTPFLEGEMITTEL
ENSEMBLE DE COUSSINET POUR SEIN CONTENANT UN INGREDIENT BON POUR LA PEAU

(30) Priority: 30.11.2001 US 998500
(43) Date of publication of application: 25.08.2004
(73) Proprietor: KIMBERLY-CLARK WORLDWIDE, INC., Neenah, Wisconsin 54956 (US)
(72) Inventor: LANGE, Beth Anne, Germantown, Tennessee 38139 (US); TYRRELL, David John, Appleton, WI 54911 (US); KRZYSIK, Duane, Gerard, Appleton, WI 54911 (US); LAABS, John, Edward, Hortonville, WI 54944-9334 (US); WILLIAMSON, Bruce, Scott, Alpharetta, GA 30005 (US)
(74) Representative: Beacham, Annabel Rose
(86) International application number: PCT/US2002/026703
(87) International publication number: WO 2003/047644

(56) References cited:
- WO-A-97/01994
- DE-A- 4 432 633
- US-A- 5 013 569
- US-A- 5 709 855

## Description

The present invention relates to a breast pad assembly suitable for absorbing lacteal fluid which is comfortable to the human breast. More particularly, the present invention is directed to a disposable or re-washable breast pad assembly suitable for absorbing lacteal fluid which is comfortable to the human breast and contains a composition comprising a skin benefit ingredient such as a lipid comprising omega-3 fatty acids and/or essential fatty acids.

During pregnancy, human breasts enlarge under the stimulation of hormones generated by the female body. Once the placenta is delivered, milk may be secreted from the nipples of the breast. Milk secretion from the breast is initiated by prolactin and is maintained by nipple-sucking stimulation. During the period of time breast feeding is maintained, the breasts and nipples are highly sensitive and may secrete milk even when suckling is not occurring.

One possible complication of breast feeding after delivery that may lead the mother to discontinue lactation is cracked and painful nipples. During breast feeding, the nipple may become painful, sore, sting, and show fissures and ulcers. Cracked and painful nipples may be caused, in part, by the loss of naturally occurring lipids on the skin surface which may be lost during breast feeding. Due to skin lipid loss, the nipples and areolas may appear inflamed, bright pink, and/or may flake and peel. A decrease in the amount of breast milk moving through the nipple may cause the baby to suck energetically, bringing about nipple erosions. Several types of infections may also cause nipples to crack. *Candida albicans* is frequently implicated in the infection of cracked, painful nipples in lactating women. Currently, typical treatment regimens may include topical antiseptics, Nystatin cream, ointments, or oral anti-fungals if lactation is discontinued.

Another complication from breast feeding is acute lactation mastitis which is an infectious breast disease that occurs in about 20% of lactating women. Tenderness of the nipple and/or breast is an initial symptom, and is usually accompanied by erythema, heat and edema. Breast feeding is encouraged as part of the treatment for mastitis but is often uncomfortable for the mother. Failure to tolerate frequent breast emptying may lead to early discontinuation of breast feeding. Oral antibiotics and/or over the counter drugs are typically used for treating mastitis, leading to exposure of the infant to the antibiotic or drug also. Failure to aggressively treat mastitis can result in an ascending infection of the lactiferous duct and more serious breast infection.

Devices for preventing breast milk leakage from contacting and seeping into and through clothing and bed linens are currently commercially available. Generally, these types of devices fall into two broad categories: nursing or breast pads and nursing or protective brassieres. These two categories each comprise two general subcategories, reusable pads and disposable pads, and unitary brassieres and brassieres having replaceable absorbent pads. Although these devices are typically satisfactory for absorbing leaking breast milk and thereby keeping the milk from soaking through a woman's clothes or staining bed linens, currently available breast pads are not sufficiently capable of preventing or treating other problems associated with breast feeding such as nipple tenderness, nipple cracking and/or acute lactation mastitis. In addition, the breast pad itself as currently available may induce further frictional damage to the skin. As such, a need exists in the marketplace for improved breast pads capable of enhancing nipple and breast skin health while still having the ability to prevent breast milk leakage. Additionally, it would be highly beneficial if the breast pad could enhance nipple and breast skin health while simultaneously improving the health of the baby.

WO97/01994 discloses a breast pad, the skin contacting side of which comprises a therapeutically active composition. No omega-3 fatty acids are disclosed. US5,709,855 discloses a topical therapeutic composition comprising a mixture of omega-3 and omega-6 fatty acids. DE-A 4432633 discloses a topical therapeutic composition comprising omega-3 fatty acids. US5,013,569 discloses an infant food formulation comprising a mixture of omega-3 fatty acids.

### SUMMARY OF THE INVENTION

The present invention provides breast pads incorporating a composition comprising a skin benefit ingredient for use by expectant and/or nursing mothers whose breasts have enlarged due to the presence of milk. During breast feeding, a mother can lose a significant amount of lipids from the breast and nipple skin resulting in inflammation, redness and potential infection to the breast and/or nipple. In accordance with the present invention, a composition comprising a lipid comprising omega-3 fatty acids is introduced onto the surface of the breast pad which faces the mother during use such that the omega-3 fatty acids can be transferred from the breast pad to the mother's breast skin and nipple during use to replenish skin lipids lost from the breast and nipple skin during breast feeding. Along with providing a skin health benefit to the mother by replacing lost breast and nipple skin lipids and thereby improving skin barrier functions, omega-3 fatty acids can be ingested by the suckling baby to increase the amount of omega-3 fatty acids in the baby's diet to improve health.

In another embodiment of the present invention, the lipid comprises an essential fatty acid which provides a skin health benefit to the mother and can also be ingested by the baby during breast feeding for improved health and development of the baby's nervous and immune system. Further, in another embodiment, the lipid incorporated onto the breast pad may be comprised of both omega-3 fatty acids and essential fatty acids. This combination of both omega-3 fatty acids and essential fatty acids may be highly beneficial to both the mother and the baby simultaneously.

Briefly, therefore, the present invention is directed to a breast pad for absorbing fluid leaking from a breast of a woman and minimizing the soiling of clothing worn by the woman. The breast pad has a front side which faces the breast and a back side which faces the clothing. The front side of the breast pad comprises from 1.0g/m² to 30 g/m² of a composition for improving breast and nipple skin health. The composition comprises omega-3 fatty acids.

The composition may further comprise omega-6 fatty acids.

Preferably, the composition comprises from 1% (by total weight of the composition) to 15% (by total weight of the composition) of flaxseed oil.

Also preferably, the composition comprises linoleic acid, alpha linoleic acid, eicosapentenoic acid, and decosahexenoic acid.

The breast pad of the invention may be used for treating or preventing nipple tenderness and cracking on the breast of breast feeding woman. The composition is introduced onto a front side of a breast pad to be worn by the woman. The breast pad comprises a front side which faces the wearer and a back side which faces the clothing. The composition introduced onto the breast pad comprises omega-3 fatty acids. The composition is transferred from the breast pad to the nipple and breast of the woman during wear such that the omega-3 fatty acids contact the nipple of the breast and replace skin lipids lost by the mother during the breast feeding.

The invention is further directed to a method of supplementing the nutrient intake of a breast feeding infant. The method comprises first introducing a composition onto a front side of a breast pad to be worn by the woman. The breast pad comprises a front side which faces the wearer and a back side which faces the clothing. The composition introduced onto the breast pad comprises omega-3 fatty acids. The composition is transferred from the breast pad to the nipple and breast of the woman during wear such that the omega-3 fatty acids contact the nipple of the breast. Finally, the omega-3 fatty acids are transferred from the nipple and breast of the woman to the infant during breast feeding.

Other objects and features of this invention will be in part apparent and in part pointed out hereinafter.

### DETAILED DESCRIPTION OF THE PREFERRED EMBODIMENTS

In accordance with the present invention, it has been discovered that breast and nipple infection and nipple cracking and peeling and nipple fissures and ulcers caused by breast feeding and infection after delivery can be substantially minimized or eliminated by introducing a composition comprising a lipid onto a breast pad worn by the woman to control unwanted leakage of breast milk. The breast pad can be used as a skin wellness system to supply natural ingredients, such as lipids rich in omega-3 fatty acids, to the nipple and breast skin that are lost during breast feeding. Surprisingly, lipids containing high concentrations of omega-3 fatty acids provide a significant skin health benefit to the nipple and breast skin of the woman by enhancing skin barrier properties, providing anti-inflammatory and antimicrobial activity, and by reducing friction between the breast pad and the nipple and breast skin during use of the breast pad. Further, ingestion of the omega-3 fatty acids by the nursing child may lead to numerous health benefits. Along with lipids having a high concentration of omega-3 fatty acids, other lipids having a high concentration of omega-6 fatty acids and/or essential fatty acids can be introduced onto the breast pad in a suitable composition to improve the skin health of the mother and the health of the suckling baby. Additionally, other additives such as natural moisturizing factors or humectants can be used in combination with the lipids described herein to provide additional benefits to the breast and nipple skin of the mother, and potentially improve the health of the baby.

In accordance with the present invention, there is provided a breast pad or nursing pad containing a composition comprising a skin health benefit ingredient. The breast pad is used as a skin wellness system to supply skin supplements to the breast skin and nipple that may be lost from the breast and nipple during breast feeding, and may also indirectly supply important fatty acids to the nursing child. As used herein, the terms "breast pad" or "nursing pad" are intended to mean either disposable or re-washable pads, liners, or inserts for use with or without a brassiere to absorb leakage from a woman's breast such that the clothes or bed linens of the woman are not soiled. Breast pads are known in the art and typically are comprised of at least three distinct layers. The first layer, which generally faces the breast skin and nipple, is typically comprised of a wicking material for wicking moisture away from the breast to a second layer. The second layer is typically a highly absorbent layer which accepts moisture from the wicking material. This second layer is where the moisture is typically held. The third layer, which typically faces the clothes of the wearer, or the brassier, is generally comprised of a moisture resistant layer to keep the outer clothing or brassier dry. As one skilled in the art will recognize, there are numerous types and styles of breast pads available and all can be used in accordance with the skin benefit ingredients described herein.

In one embodiment of the present invention, a composition comprising a lipid is introduced onto the face of a breast pad facing and in contact with the woman's breast. As used herein, the term "lipid" includes, but is not limited to, fats and oils and their acid analogues (i.e., fatty acids). As mentioned above, a significant amount of breast skin lipids may be lost from the outer layer of the skin, the stratum corneum, during breast feeding by the mother. The loss of these breast skin lipids, which are required for proper skin barrier functions which protect against water loss and infection, along with other events which occur during breast feeding such as nipple and breast hydration, can lead to a compromised stratum corneum and subsequent problems with the breast skin and nipple including cracking, peeling, soreness, ulcers and infection.

In accordance with the present invention, compositions containing lipids are introduced onto a breast pad and transferred to the breast skin and nipple during normal wear and use to replenish the natural supply of skin lipids and improve the health of the breast and nipple skin. Because breast pads are typically worn by nursing mothers for a substantial period most days during nursing, the breast pads of the present invention can consistently supply lipids, and possibly other compounds, to the breast and nipple skin to help repair and protect damaged skin. Lipids suitable for introduction onto the breast pad of the present invention can be found in numerous oils including, for example, fish oil, olive oil, canola oil, walnut oil, flaxseed oil, cottonseed oil, avocado oil, lanolin, safflower oil, soybean oil, and sunflower oil. These lipid sources, as well as others, can be used alone or in combination to provide the desired concentration or combination of lipids on the breast pad. The composition comprising the lipid is introduced onto the bodyfacing surface of the breast pad such that the bodyfacing surface will contain from 0.1 grams of composition/m² of fabric to 30 grams of composition/m² of fabric. Generally, the lipid can be sprayed directly onto the bodyfacing side of the breast pad, or can be immobilized on the surface of the bodyfacing side of the breast pad to produce the intended benefit.

When introduced onto the breast pad, the lipid is typically formulated into a liquid (for spraying) or a solid (for solid immobilization) composition which is introduced onto a portion or the entire bodyfacing surface of the breast pad. The composition can be solid or semisolid in accordance with the present invention, and may, for example, contain petrolatum, or other additives as discussed herein, to improve formulability and other aspects of composition. The composition may be in a variety of forms, including, but not limited to, emulsions, lotions, creams, ointments, salves, suspensions, encapsulations, gels, and the like. The composition can be applied to the bodyfacing side of the breast pad using a variety of techniques including foam application, spraying, slot coating and printing. The present invention also encompasses technology that would permit integration of the composition directly with fibers or other materials used to form the breast pad. As mentioned above, the compositions can be applied to the bodyfacing surface of the breast pad in amounts of from 0.1 g/m² of breast pad fabric to 30 g/m² of breast pad fabric.

Compositions, such as those containing petrolatum along with the lipid, are typically applied to the bodyfacing material of the breast pad during manufacture. In order to process and apply the formulations to the bodyfacing materials, it is preferred that the formulations be in a semi-solid or fluid state. However, in order to have stability on the bodyfacing material after manufacture, the formulations need to be semi-solid or solid across a wide range of shipping and storage temperatures. The lipid containing compositions of the present invention may also include from about 1% (by total weight of the composition) to about 99% (by total weight of the composition) of one or more solidifying agents. More specifically, the compositions may include from about 25% (by total weight of the composition) to about 75% (by total weight of the composition) of solidifying agents. Even more specifically, the compositions may include from 40% (by total weight of the composition) to 60% (by total weight of the composition) of solidifying agents. Solidifying agents are capable of solidifying the composition so that the composition is solid at room temperature and has a penetration hardness of at least about 5 millimeters. More specifically, the solidifying agents include one or more materials that are capable of solidifying the natural fats/oils and emollient combination so as to have a penetration hardness of from about 5 millimeters to about 365 millimeters at 25°C. Further, the solidifying agents solidify the emollient (or the fats/oils/emollients combinations when fats and oils are used in the composition) so that it has a melting point between about 32°C and about 100°C. One or more solidifying agents can be selected from alkyl siloxanes having a melting point greater than about 35°C, polymers, waxes (animal, vegetable or mineral), glycerides (mono-, di-, and tri-) having a melting point greater than about 35°C, hydrogenated vegetable and/or animal oils having a melting point of 35°C or greater, fatty acid esters having a melting point greater than about 35°C or greater, and branched esters. Examples of suitable solidifying agents include, but are not limited to, the following compounds: C₁₆ and greater alkyl silicones, beeswax, behenyl behenate, candelilla wax, carnauba, synthetic carnauba, PEG-12 carnauba, cerasin, hydrogenate microcrystalline wax, jojoba wax, microcrystalline wax, lanolin wax, ozokerite, paraffin, synthetic paraffin, cetyl esters, C₂₀-C₄₀ alkyl behenate, C₁₂-C₁₅ lactate, cetyl palmitate, stearyl palmitate, isosteryl behenate, lauryl behenate, behenyl isostearate, cetyl myristate, cetyl octanoate, cetyl oleate, cetyl ricinoleate, cetyl stearate, decyl oleate, myristyl lactate, myristyl lignocerate, myristyl myristate, myristyl stearate, lauryl stearate, octyldodecyl stearate, octyldodecyl stearoyl stearate, oleyl arachidate, oleyl stearate, tridecyl behenate, tridecyl stearate, tridecyl stearoyl stearate, pentaerythrityl teterbehenate, pentaerythritylhydrogenated rosinate, pentaerythrityl distearate, pentaerythrityl tetraabeite, pentaerythrityl tetracocate, pentaerythrityl tetraperlargonate, pentaerythrityl tetrastearate, polyethylene, hydrogenated cottonseed oil, hydrogenated vegetable oil, hydrogenated squalene, hydrogenated coconut oil, hydrogenated castor oil, hydrogenated jojoba oil, hydrogenated palm oil, hydrogenated palm kernel oil, hydrogenated olive oil, polyamides, C₃₀-C₆₀ fatty alcohols, polypropylene, polybutylene terephthalate, dodecyl laurate, stearyl palmitate, octadecyl hexadecanoate, octadecyl palmitate, stearyl behenate, docosyl octanoate, tetradecyl-octadecanyl behenate, hexadecyl-cosanyl hexacosanate, shellac wax, glycol montanate, fluoranated waxes, and mixtures of such compounds.

The lipid containing compositions of the present invention may also include from 1% (by total weight of the composition) to 40% (by total weight of the composition) of one or more fatty alcohols which help to provide the compositions of the present invention in solid form. As such, fatty alcohols act to assist in maintaining and stabilizing the compositions on the bodyfacing surface, as well as acting as an emollient. More specifically, the compositions may include from 10% (by total weight of the composition) to 25% (by total weight of the composition) of fatty alcohols. As used herein, suitable fatty alcohols include, but are not limited to, the following materials: alcohols having a carbon chain length of C₁₄-C₃₀ or greater, including cetyl alcohol, stearyl alcohol, arachidyl alcohol, behenyl alcohol, and mixtures thereof

The lipid containing compositions of the present invention may also include sterols, sterol derivatives or mixtures of both which can act in combination with naturally occurring fats and oils and/or the lipids of the present invention to provide skin barrier enhancement and skin barrier recovery. Typical amounts can be from 0.1 % (by total weight of the composition) to 10% (by total weight of the composition). Sterols and sterol derivatives include compounds such as beta-sterols with a tail on the 17 position and no polar groups, such as cholesterol, C₁₀-C₃₀ cholesterol/lanosterol esters, tall oil sterols, soy sterols, sterol esters and mixtures of these compounds. More specifically, the composition of the present invention may include from 0.5% (by total weight of the composition) to 5% (by total weight of the composition) of sterols, sterol derivatives or mixtures of both. Examples of suitable sterol compounds include cholesterol, sitosterol, stigmasterol, and ergosterol, as well as C₁₀-C₃₀ cholesterol/lanosterol esters, cholecalciferol, cholesteryl hydroxystearate, cholesteryl isostearate, cholesteryl stearate, 7-dihydrocholesterol, dihydrocholesterol, dihydrocholesteryl octyldecanoate, dihydrolanosterol, dihydrolanosteryl octyldecanoate, ergocalciferol, tall oil sterol, soy sterol acetate, lanasterol, soy sterol, avocado sterols, sterol esters and mixtures thereof.

The lipid containing compositions of the present invention may also include from 0.1 % (by total weight of the composition) to 10% (by total weight of the composition) of one or more extracted botanicals. More specifically, the compositions may include from 0.5% (by total weight of the composition) to 5% (by total weight of the composition) of one or more extracted botanical actives. The extracted botanical actives, in combination with the other components of the composition, provide several benefits to the skin, particularly skin that is frequently covered by an absorbent article. The extracted botanical actives in combination with other components of the composition such as the lipids, can act as anti-irritants and antioxidants on the skin's surface. Significantly, the botanical extracts may also prevent the oxidation of the lipids of the present invention in the composition, thus allowing the lipids to maintain effectiveness. Extracted botanical actives can include any water-soluble or oil-soluble active extracted from a particular plant. Examples of suitable extracted botanical actives are actives extracted from echinacea, yucca glauca, grape seed extract, willow herb, basil leaves, oregano, carrot root, grapefruit fruit, fennel fruit, rosemary, thyme, blueberry, bell pepper, black tea, blackberry, black currant fruit, Chinese tea, dandelion root, date palm fruit, gingko leaf, green tea polyphenols (including epicatechin gallate and epigallocatechin 3-O-gallate), hawthorn berries, licorice, oolong tea, sage, strawberry, sweet pea, tomato, vanilla fruit, neohesperidin, quercetin, rutin, morin, myricetin, chlorogenic acid, glutathione, glycyrrhizin, centella asiatica, chamomile, comfrey, cornflower, horse chestnut, ivy (*Herdera helix*), magnolia, mimosa, oat extract, pansey, seabuckthorn, white nettle, witch hazel, and any combinations thereof. Botanicals are primarily extracts of the plants from which the originate and botanicals are available from suppliers as part of a composition that also contains an extracting solvent. Amounts of the botanicals in the compositions of the present invention in terms of active component (not extract) may range from about 0.000001% (by total weight of the composition) to 10% (by total weight of the composition). Desirably, the amount of active botanical is from about 0.00001% (by total weight of the composition) to about 5% (by total weight of the composition) and more desirably from about 0.001% (by total weight of the composition) to about 1% (by total weight of the composition). Further, it is also desirable that the amount of active botanical is from about 0.001% (by total weight of the composition) to about 0.5% (by total weight of the composition), and more desirably from about 0.001% (by total weight of the composition) to about 0.1% (by total weight of the composition).

The lipid containing compositions of the present invention may further include from 0.1% (by total weight of the composition) to 10% (by total weight of the composition) of one or more emollients to help maintain the soft, smooth and pliable nature of skin. The emollients have the ability to remain on the skin surface in the stratum corneum to act as lubricants to reduce flaking and improve the skin's appearance. More specifically, the compositions may include from 0.5% (by total weight of the composition) to 5% (by total weight of the composition) of emollient(s). Even more specifically, the compositions of the present invention may include from 1% (by total weight of the composition) to 5% (by total weight of the composition) of emollient(s). Suitable emollients include petroleum based oils, petrolatum, vegetable oils, mineral oils, lanolin and its derivatives, fatty esters, glycerol, glycerol esters and their derivatives, propylene glycol esters and their derivatives, alkoxylated carboxylic acids, alkyoxylated alcohols, fatty alcohols, alkyl methicones, alkyl dimethicones, phenyl silicones, alkyl trimethicones, dimethicone and mixtures of such compounds. The dimethicone can be blended with the other components through the addition of water-based emulsions containing dimethicone.

Optionally, the lipid containing compositions of the present invention may include from 1% (by total weight of the composition) to 20% (by total weight of the composition) of one or more viscosity enhancers. The viscosity enhancers can be added to increase the melt point viscosity of the compositions. Increasing the melt point viscosity gives better stability of the compositions on the bodyfacing materials of the articles. The viscosity enhancers also improve the stability of the compositions at the stability temperature of about 54.5°C (130°F). Having high viscosity >50.0 Pa·s (>50,000 centipoise) at elevated temperatures prevents the compositions from migrating into or away from the materials to which they are applied. However, the viscosity enhancer component also provides a low viscosity <5.0 Pa·s (<5,000 centipoise) for the compositions under high shear and at processing temperatures. The viscosity enhances suitable for use with the present invention are capable of providing a desirable viscosity to the compositions of the present invention, depending on shear and temperature conditions. The viscosity enhancers are suitable for use for many compositions having a range of melting points. While it is desirable for compositions of the present invention to have increased viscosity under "hot box car" stability conditions, the increased viscosity can be maintained, in part, through the use of one or more viscosity enhancers up to the melting point of the particular compositions. Typically, process temperatures are approximately 5°C above the melting point of the composition. Examples of suitable viscosity enhancers for use in combination with the compositions and products of the present invention include, for example, polyolefin resins, lipophilic/oil thickeners, ethylene/vinyl acetate copolymers, organically modified clays, polyethylene, silica, silica silylate, silica methyl silylate, colloidal silicone dioxide, alkyl hydroxy ethyl cellulose, other organically modified celluloses, glycol montanate, PEG-12 carnauba, C₂₀-C₄₀ alkyl hydroxystearyl stearate, polyperfluoromethylisopropylether montan wax, and mixtures of these compounds. Many of the solidifying agents described herein have also been found to provide the same benefits to the compositions described herein as the viscosity enhancers.

In addition to one or more viscosity enhancers, the compositions of the present lipid-containing compositions of the present invention may also include one or more rheology modifiers. Rheology modifiers are compounds that increase the viscosity of the compositions at lower temperatures as well as process temperatures. Rheology modifiers or suspending agents also provide "structure" to the compositions to prevent settling out (separation) of insoluble and partially soluble components. Other components or additives of the compositions may effect the temperature, viscosities, and rheologies of the compositions. By increasing the viscosity at process temperatures, the rheology modifiers will increase the low shear viscosity about 50.0 Pa·s (50,000 centipoise). However, the rheology modifiers are thixotropic in behavior; therefore, their viscosity decreases as shear and pressure increases. Consequently, when the rheology modifiers are used in the compositions described herein, they maintain the suspension of insoluble and partially soluble components. This capability can be particularly important if, during processing, the composition must be left stagnant in process lines,and hoses. The rheology modifiers will maintain the suspension of the insoluble and partially soluble components for a period of time that depends on the viscosity of the composition and on the amount of rheology modifier present. The thixotropic behavior of the rheology modifiers causes their viscosity to drop when processing is resumed and the composition is no longer stagnant due to the application of pressure and shear forces. In addition to stabilizing the suspension of insoluble and partially soluble components, the rheology modifiers of the invention also help to stabilize the compositions on the bodyfacing or other materials to which the compositions are applied. Examples of suitable rheology modifiers include silica, silica silylate, silica methyl silylate, quaternary starch compounds, quaternary modified clays, organically modified clays, and mixtures thereof. Such rheology modifiers can help maintain the suspension of an insoluble emollient, such as a siloxane, or particulates such as microencapsulates, anionic polymers, clays and inorganic materials, within the compositions. The compositions described herein can include from 0.5% (by total weight of the composition) to 20% (by total weight of the composition) and more preferably from 0.5% (by total weight of the composition) to 10% (by total weight of the compositon) of one or more rheology modifiers.

The use of a rheology modifier or more than one rheology modifier, such as an organically modified clay in combination with silica, can provide a benefit to the rheology of the compositions of the present invention by increasing the viscosity of the compositions at process temperatures. When a silica, an organically modified clay or both are used in an ointment or lotion type composition, it is expected that they will increase the hardness of the composition and, consequently, have a potentially negative effect on transfer of the ointment or lotion to the skin. However, when a natural clay or a synthetic analog of a natural clay is used in combination with an organically modified clay or a silica, there is an unexpected, synergistic enhancement of the rheology of the composition. The organically modified clay and silica assist the suspension of the natural clays/synthetic analog of a natural clay in the compositions of the present invention. While the penetration hardness of the composition increases, the transfer of the composition is not affected due to rheology enhancement and, in many instances, the transfer is increased. When a natural clay or synthetic analog of a natural clay is combined with an organically modified clay or a silica, a small amount of shear (such as rubbing) will unexpectedly cause the composition to become soft and spread easily. Therefore, when such a combination is used in the compositions of the present invention, there is an improvement in the transfer of the composition from the bodyfacing surface to the nipple and breast skin. Though these composition provide improved transfer from the product to the nipple and breast skin, they remain stable on the product surface under storage conditions. Natural clays include montmorillonite, bentonite, beidellite, hectorite, saponite, stevensite, magnesium aluminum silicate and similar clays. Synthetic analogs of natural clays, such as laponite synthetic clay available from Southern Clay Products, Inc. of Gonzales, Texas can also be used to provide the rheology benefit to compositions described herein when used in combination with organically modified clays or silica.

The lipid containing compositions of the present invention typically have a melting point of from about 32°C to about 100°C. Melting behavior in this range provides compositions that are relatively immobile and localized on the bodyfacing surface of the breast pads of the present invention. Though relatively immobile and localized at room temperature, the compositions are also readily transferable to the wearer of the breast pad at body temperature through natural rubbing or friction during wearing and/or through adhesion of the lipid containing composition to the skin of the wearer. The compositions also maintain their integrity and are not completely liquid at elevated temperatures such as may be experienced during storage. Stability in a solid state at elevated temperatures is made possible, in part, by the melting point and structure provided by the solidifying agent and the addition of viscosity enhancers and rheology modifiers, if needed, in the formulation. Desirably, the composition of the invention are easily transferable to the skin by way of normal contact, including adhesion of the composition to the skin, wearer motion and/or body heat. Because the compositions are relatively immobilized on the bodyfacing surface of the breast pad, the quantities of the compositions necessary to provide the desired skin barrier benefits are reduced. In addition, special barrier or wrapping materials may not be necessary for the breast pads of the present invention.

The compositions of the present invention have high shear viscosities of less than about 5.0 Pa·s (5,000 centipoise) at processing temperatures such as at a temperature of about 60°C or higher. The melting points and, therefore, the processing temperatures vary for different compositions of the invention. At about 55°C or less the compositions have low shear viscosities greater than about 50.0 Pa·s (50,000 centipoise). The compositions can have process viscosities of less than about 0.1 Pa·s (100 centipoise) under shear and pressure. The compositions may also have a penetration hardness of from about 5 millimeters to about 365 millimeters at 25°C.

In a preferred embodiment of the present invention, the lipid introduced onto the breast pad comprises at least some omega-3 fatty acids; that is, the lipid comprises at least some fatty acids wherein the first double bond between carbon atoms occurs three carbon atoms away from the omega end of the lipid molecule. Preferably, the lipid introduced onto the breast pad in the compositions of the present invention comprises a high concentration of omega-3 fatty acids. The three omega-3 fatty acids are alpha linolenic acid, eicosapentenoic acid, and docosahexenoic acid; all or some of which can be used alone or in combination and be incorporated onto a breast pad in accordance with the present invention to replace lipids lost during breast feeding, and provide a health benefit to the nursing baby. Preferably, the omega-3 fatty acid containing composition incorporated into the breast pad contains from 0.05% (by total weight of the composition) to 1.5% (by total weight of the composition) and more preferably from 0.1% (by total weight of the composition) to 1% (by total weight of the composition) of omega-3 fatty acids. Although amounts of omega-3 fatty acids in the composition less than about 0.05% (by total weight of the composition) are within the scope of the present invention and would provide some benefit to the nipple and breast skin, a larger amount is typically desired for improved health of the nipple and breast skin. Further, although amounts of omega-3 fatty acids in the composition greater than about 1.5% (by total weight of the composition) are within the scope of the present invention, typically such high concentrations are not required to realize the benefits of the present invention.

The omega-3 fatty acids on the breast pad enhance nipple and breast skin health by replacing lipids lost from nipple and breast skin during breast feeding. Further, the omega-3 fatty acids comprising the lipid may also provide some anti-inflammatory and antimicrobial benefit to the nipple and breast skin further reducing the chance of infection and/or irritation, and further improving the health of the breast and nipple skin. As such, omega-3 fatty acids, when introduced onto the breast and nipple skin surface through interaction with the breast pad, may reduce swelling of the nipple and breast and may control the growth of unwanted microbes which can lead to infection or ultimately be transferred from the nursing mother to the baby during breast feeding. Significantly, because breast pads are generally worn by the woman to control leakage as soon as milk is delivered to the breast, lipids are continuously replaced on the nipple and breast early in, and throughout, the breast feeding cycle, which can reduce the possibility of the woman having significant breast or nipple discomfort or infection, which can ultimately lead to the discontinuation of nursing.

Along with the significant benefits lipids comprising omega-3 fatty acids can impart on the breast and nipple skin of the mother, these omega-3 fatty acids can also be ingested by a baby during breast feeding and lead to improved health for the child as the omega-3 fatty acids are on the breast and nipple skin of the mother. It is known that omega-3 fatty acids and their derivatives are essential for the proper development of a baby's nervous and immune systems, and may also directly affect the cardiovascular system. It is also well-known that many babies can be deficient in omega-3 fatty acids, which can in turn negatively effect the development of these critical systems in the baby's body. By incorporating lipids containing omega-3 fatty acids onto the breast pad of the present invention such that some of the fatty acids are transferred onto the mother's nipple and breast, along with the skin benefits obtained by the mother, the child can ingest some of the omega-3 fatty acids with the mother's milk during breast feeding, thereby increasing the amount of omega-3 fatty acids available in the child's body resulting in improved health.

Numerous sources of lipids comprising omega-3 fatty acids are known and can be used in combination with a breast pad within the scope of the present invention. Specifically, fish oil, olive oil, canola oil, walnut oil and flaxseed oil contain a particularly high concentration of omega-3 fatty acids and can be introduced in suitable composition onto the breast pads of the present invention to produce the desired benefits. A particularly preferred source of omega-3 fatty acids is flaxseed oil, which is the richest source of omega-3 fatty acids, and contains from about 55% to about 65% alpha linoleic acid. Also, combinations of any one or more of the above-noted omega-3 fatty acid sources can be utilized in combination with the breast pad of the present invention to produce the intended benefits.

Numerous lipids which can be introduced onto the breast pad of the present invention comprise omega-6 fatty acids in addition to omega-3 fatty acids. Omega-6 fatty acids are also beneficial in replenishing breast and nipple skin lipids lost during the breast feeding process. For example, corn oil, cottonseed oil, safflower oil, and sunflower contain relatively high percentages of omega-6 fatty acids; these sources can be used in combination with omega-3 fatty acid sources in accordance with the present invention. Many lipids suitable for introduction onto the breast pad of the present invention comprise a high percentage of both omega-3 fatty acids and omega-6 fatty acids. Both of these fatty acids are suitable for use on the breast pad of the present invention for replenishing breast and nipple skin lipids lost during the breast feeding process.

Omega-3 fatty acids and omega-6 fatty acids compete in the metabolic pathway of humans. As such, too high a concentration of omega-6 fatty acids present in the body can inhibit the metabolism of omega-3 fatty acids which are critical for good health. Because both omega-3 and omega-6 fatty acids may be transferred from the breast pad to the breast and nipple skin and may be ultimately ingested by a baby during breast feeding, it is preferred that when omega-6 fatty acids are introduced onto the breast pad in combination with omega-3 fatty acids, that the ratio of omega-3 fatty acids to omega-6 fatty acids be controlled. If too much omega-6 fatty acid is ingested into the baby and not enough omega-3 fatty acid is present, the baby may be subjected to various forms of inflamation or other unwanted problems. As such, when lipids comprising both omega-3 fatty acids and omega-6 fatty acids are introduced onto the breast pad in a composition in accordance with the present invention, it is preferred that the weight ratio in the composition of omega-3 fatty acids to omega-6 fatty acids be from 1:2 to 1:4, respectively. Such a weight ratio will not only replenish skin lipids on the mother's breast and nipple skin, but may also be safely ingested by the baby and significantly benefit the health of the baby by supplying a healthful ratio of omega-3 and omega-6 fatty acids.

In another embodiment of the present invention, lipids comprising essential fatty acids can be introduced onto a face of a breast pad which faces the breast during wear to improve the health of the woman's breast skin and nipple and potentially improve the health of the suckling baby. As used herein, the term "essential fatty acids" means fatty acids which cannot be synthesized by the human body and must be obtained from a dietary source. Because humans lack the required enzyme to introduce carbon-carbon double bonds at carbon atoms beyond the ninth carbon atom in unsaturated fatty acids (the ninth carbon atom from the omega end of the chain), linoleic acid (an omega-6 fatty acid) and alpha linolenic acid (an omega-3 fatty acid) are essential fatty acids that must be obtained by humans from a dietary source to ensure good heath. Many humans have been found to be deficient in essential fatty acids which can lead to numerous heath ailments and problems.

On human skin, the essential fatty acids linoleic acid and alpha linolenic acid can be chemically altered to monohydroxy fatty acids. It is known that the enzyme epidermal 15-lipoxygenase can chemically alter essential fatty acids and their derivatives to monohydroxy fatty acids. For example, this skin enzyme chemically alters dihomo-gammalinolenic acid to 15-hydroxyeicosatrienoic acid, eicosapentaenoic acid to 15-hydroxyeicosapentaenoic acid, and docosahexaenoic acid to 17-hydroxydocosahexaenoic acid. These monohydroxy acid analogues of the essential fatty acids exhibit anti-inflammatory properties in vitro by modulating the secretion of inflammatory eicosanoids and cytokines by competitive inhibition of the eicosanoids synthase systems. Also, these analogues may exhibit antimicrobial activity on the skin's surface. As such, by introducing essential fatty acids onto the face of a breast pad which faces the breast of a woman during use, the fatty acids can replenish lost skin lipids and any monohydroxy fatty acid analogues generated on the skin's surface from these essential fatty acids may generate a local cutaneous anti-inflammatory effect and antimicrobial on the breast skin and nipple of the woman. This anti-inflammatory effect may serve as an improved alternative to common in vivo mono-therapies currently utilized as the ingestion by the suckling baby of these essential fatty acids or their analogues will not be harmful to the baby, and may supply essential fatty acids to the baby to improve health. Further, the essential fatty acids could be used in combination with standard skin protectants such as petrolatum and therapeutic regimes such as hydrocortisone for the management of inflammatory skin disorders.

In accordance with the present invention, it is preferred that the lipid comprising the essential fatty acid to be introduced onto the breast pad contain at least about 1% essential fatty acid, more preferably at least about 5% essential fatty acid, and more preferably at least about 10% essential fatty acid. Sources (including both seeds and oils) containing linoleic acid include safflower, sunflower, walnut, sesame, hemp, grape, peanut, evening primrose, chia, kukui or candlenut, canola, soybean, wheat germ, flax, rice bran, corn and olive. Sources (including both seeds and oils) containing alpha linoleic acid include flax, chia, kukui or candlenut, hemp, pumpkin, walnut, and soybean. Preferably, the essential fatty acid containing composition incorporated into the breast pad contains from about 0.05% (by total weight of the composition) to about 1.5% (by total weight of the composition), more preferably from about 0.1% (by total weight of the composition) to about 1% (by total weight of the composition) of essential fatty acids. Although amounts of essential fatty acids less than about 0.05% (by total weight of the composition) are within the scope of the present invention and would provide some benefit to the nipple and breast skin, a larger amount is typically desired for improved health of the nipple and breast skin.

In an alternative embodiment of the present invention, lipids comprising omega-3 fatty acids and essential fatty acids can be introduced onto the face of a breast pad facing and in contact with the woman's breast. Optionally, omega-6 fatty acids may also be introduced onto the breast pad with this embodiment. In this embodiment, both omega-3 fatty acids and essential fatty acids act to replenish lost breast and nipple skin lipids as described above and also can be ingested by a suckling baby to improve the baby's health. If omega-6 fatty acids are introduced onto the breast pad along with the omega-3 fatty acids and essential fatty acids, as mentioned above it is preferred that the weight ratio of omega-3 fatty acids to omega-6 fatty acids be 1:2 to 1:4.

Along with the lipids described herein which may be introduced onto a breast pad in accordance with the present invention to improve the health of the mother's breast and nipple skin and potentially the health of the baby, other additives may be introduced onto the breast pad in combination with the lipids described herein to further improve the breast and nipple skin health of the woman and possibly the health of the baby if such additives are ingested by the baby during breast feeding. In one embodiment, a natural moisturizing factor additive can be introduced onto the breast pad in addition to the lipids described above to increase nipple and breast skin softness and suppleness and improve nipple and breast skin elasticity. For example natural moisturizing factors such as sodium pyrrolidone carboxylic acid, urea, lactic acid, glycolic acid, phopholipids, malic acid, pyrivic acid or combinations could be used in combination with the lipid containing breast pads of the present invention. Edible humectants such as sugars and sugar alcohols including mannitol, maltose, lactose, dextrose, sucrose, sorbitol, fructose, and honey could be used in combination with the lipids described herein to improve the overall breast and nipple skin health of the mother.

In another embodiment of the present invention, other additives such as vitamin E, vitamin C, humectants, and aloe may also be used in combination with the lipids and natural moisturizing factors described herein to improve the breast and nipple skin health and potentially improve the health of the baby if ingested. For example, algae extract can be used in combination with the lipids described herein as a suitable humectant to improve the softness of the breast and nipple skin to facilitate breast feeding. Further, alpha hydroxy acids such as glycolic acid, lactic acid, and mixed fruit acids, can be used in combination with the lipids described herein as a suitable humectant to improve the skin of the breast and nipple. Along with providing a benefit to the mother's nipple and/or breast skin, it is important that any additives introduced onto the breast pad in combination with the lipids described herein not be harmful to a baby if ingested by the baby during breast feeding. The present invention thus provides a dual benefit in that it helps to maintain the breast and nipple skin of the mother and can also provide a dietary benefit to the suckling baby if ingested during breast feeding.

The additives as described herein that can be used in combination with the lipid-containing breast pad of the present invention are typically present on the breast pad in an amount sufficient to provide their intended benefit. Typically, an amount of from 1% (by total weight of the composition) to 15% (by total weight of the composition), preferably from 1% (by total weight of the composition) to 10% (by total weight of the composition) of the additive in the composition introduced onto the breast pad is preferred. In order to better enhance the benefits to the wearer of the breast pad, and potentially to the nursing baby, additional ingredients can be included in the compositions of the present invention. For example, the classes of ingredients that may be used and their corresponding benefits include, without limitation: antifoaming agents (reduce the tendency of foaming during processing); antimicrobial actives; antifungal actives; antiseptic actives; antioxidants (product integrity); antioxidants-cosmetic (reduce oxidation); astringents-cosmetic (induce a tightening or tingling sensation on skin); astringent-drug (a drug product that checks oozing, discharge, or bleeding when applied to skin or mucous membrane and works by coagulating protein); biological additives (enhance the performance or consumer appeal of the product); colorants (impart color to the product); deodorants (reduce or eliminate unpleasant odor and protect against the formation of malodor on body surfaces); external analgesics (a topically applied drug that has a topical analgesic, anesthetic, or antipruritic effect by depressing cutaneous sensory receptors, or that has a topical counterirritant effect by stimulating cutaneous sensory receptors); film formers (to hold active ingredients on the skin by producing a continuous film on skin upon drying); fragrances (consumer appeal); silicones/organomodified silicones (protection, water resistance, lubricity, softness); opacifiers (reduce the clarity or transparent appearance of the product); powders (enhance lubricity, oil adsorption, provide skin protection, astringency, opacity, etc.); skin conditioning agents; solvents (liquids employed to dissolve components found useful in the cosmetics or drugs); and surfactants (as cleansing agents, emulsifying agents, solubilizing agents, and suspending agents).

The skin benefit ingredients described herein which can be incorporated onto a breast pad in accordance with the present invention alone or in combination, are preferably introduced onto the breast pad in such an amount and in such a manner so as not to adversely effect, or deter a baby from, breast feeding. Specifically, the skin benefit ingredient(s) incorporated onto the breast pad preferably have a pH of from about 3 to about 8, more preferably from about 4 to about 6.5 to ensure that the pH of the ingredient(s) does not harm or adversely effect the breast and nipple skin of the mother or the suckling baby. It will be recognized by one skilled in the art that the skin benefit ingredients of the present invention can be utilized in combination with other non-antagonistic chemical agents used to control or adjust the pH of the skin benefit ingredient to an appropriate level.

The skin benefit ingredients of the present invention can be introduced onto any area of a breast pad which contacts a woman's breast or nipple such that the skin benefit ingredient(s) may be transferred from the breast pad to the breast and/or nipple skin to improve the nipple and breast skin health of the mother. The skin benefit ingredients described herein are preferably incorporated onto the breast pad in an amount such that the breast pad is not soaked or wetted to the point where the ability of the breast pad to provide its primary function of absorbing leaking breast milk from the mother's breast is significantly compromised. In some embodiments of the present invention, it is advantageous to introduce the skin benefit ingredient(s) onto at least an area of the breast pad which will be in intimate contact with the mother's nipple during use such that the skin benefit ingredient(s) will not only improve the nipple skin health by supplying lipids lost during breast feeding, but will also be ingested by the baby during nursing to potentially improve the heath of the baby.

## Claims

1. A breast pad for absorbing fluid leaking from the breast of a woman and minimizing the soiling of clothing worn by the woman, said breast pad having a front side which faces the breast and a back side which faces the clothing, said front side comprising from 0.1 g/m² to 30 g/m² of a composition for improving breast and nipple skin health, said composition comprising omega-3 fatty acids.

2. A breast pad according to claim 1, wherein said composition further comprises essential fatty acids.

3. A breast pad according to claim 1 of claim 2, wherein said composition further comprises omega-6 fatty acids.

4. The breast pad as set forth in any one of claims 1 to 3 wherein the composition comprises from 0.05% (by total weight of the composition) to 1.5% (by total weight of the composition) omega-3 fatty adds.

5. The breast pad as set forth in an one of claims 1 to 3 wherein the composition comprises from 0.1% (by total weight of the composition) to 1% (by total weight of the composition) omega-3 fatty acids.

6. The breast pad as set forth in any one of claims 1 to 5 wherein an oil selected from the group consisting of fish oil, olive oil, canola oil, walnut oil, and flaxseed oil is introduced into the composition, said oil comprising omega-3 fatty acids.

7. The breast pad as set forth in any one of claims 1 to 6 further comprising an additive selected from the group consisting of a natural moisturizing factor, a humectant, vitamin C, vitamin E, aloe, and an edible botanical.

8. The breast pad as set forth in any one of claims 1 to 7 wherein the composition has a pH of from 3 to 8.

9. The breast pad as set forth in any one of claims 1 to 8 wherein the composition further comprises from 40% (by total weight of the composition) to 60% (by total weight of the composition) of a solidifying agent.

10. The breast pad as set forth in any one of claims 1 to 9 wherein the composition further comprises from 1% (by total weight of the composition) to 40% (by total weight of the composition) of a fatty alcohol.

11. The breast pad as set forth in any one of claims 1 to 10 wherein the composition further comprises from 0.1% (by total weight of the composition) to 10% (by total weight of the composition) of an additive selected from the group consisting of sterols, sterol derivatives, and mixtures thereof.

12. The breast pad as set forth in any one of claims 1 to 11 wherein the composition further comprises from 0.1% (by total weight of the composition) to 10% (by total weight of the composition) of an extracted botanical.

13. The breast pad as set forth in any one of claims 1 to 12 wherein the composition further comprises from 0.1% (by total weight of the composition) to 10% (by total weight of the composition) of an emollient.

14. The breast pad as set forth in any one of claims 1 to 13 wherein the composition further comprises from 1% (by total weight of the composition) to 20% (by total weight of the composition) of a viscosity enhancer.

15. The breast pad as set forth in any of claims 1 to 14 wherein the composition further comprises from 0.5% (by total weight of the composition) to 20% (by total weight of the composition) of a rheology modifier.

16. The breast pad as set forth in any one of claims 1 to 14 wherein the composition further comprises from 0.5% (by total weight of the composition) to 10% (by total weight of the composition) of a rheology modifier.

17. The breast pad as set forth in claim 6 wherein the oil is flaxseed oil.

18. The breast pad as set forth in claim 3 wherein an oil selected from the group consisting of corn oil, cottonseed oil, safflower oil, and sunflower oil is introduced into the composition, said oil comprising omega-6 fatty acids.

19. The breast pad as set forth in claim 3 wherein the weight ratio of omega-3 fatty acids to omega-6 fatty acids in the composition is from 1:2 to 1:4.

20. The breast pad as set forth in claim 2 wherein the composition comprises from 0.05% (by total weight of the composition) to 1.5% (by total weight of the composition) essential fatty acids.

21. The breast pad as set forth in claim 1, wherein said composition comprises from 1% (by total weight of the composition) to 15% (by total weight of the composition) flaxseed oil.

22. The breast pad as set forth in claim 21 wherein the composition comprises from 1% (by total weight of the composition) to 10% (by total weight of the composition) of flaxseed oil.

23. The breast pad as set forth in claim 21 or claim 22 further comprising from 1% (by total weight of the composition) to 15% (by total weight of the composition) of essential fatty acids.

24. The breast pad as set forth in claim 3, wherein said composition comprises linoleic acid, alpha linoleic acid, eicosapentenoic acid, and docosahexenoic acid.

25. The breast pad as set forth in claim 24 wherein the weight ratio of omega-3 fatty acids to omega-6 fatty acids in the composition is from 1:2 to 1:4.

26. A method of supplementing the nutrient intake of a breast feeding infant, the method comprising:
introducing a composition onto a front side of a breast pad to be worn by the woman breast feeding the infant, said breast pad having a front side which faces the wearer and a back side which faces the clothing, said composition comprising omega-3 fatty acids,
transferring the composition from the front side of the breast pad to the nipple and breast of the woman during wear such that the omega-3 fatty acids contacts the nipple and breast of the woman; and
transferring the omega-3 fatty acids from the nipple and breast of the woman to the infant during the breast feeding of the infant such that the omega-3 fatty acids are ingested by the infant.

27. The method as set forth in claim 26 wherein the breast pad comprises from 0.05% (by total weight of the composition) to 1.5% (by total weight of the composition) of omega-3 fatty acids.

28. The method as set forth in claim 26 or claim 27 wherein an oil selected from the group consisting of fish oil, olive oil, canola oil, walnut oil, and flaxseed oil is introduced into the composition, said oil comprising omega-3 fatty acids.

29. The method as set forth in any one of claims 26 to 28 wherein the composition further comprises essential fatty acids.

30. The method as set forth in any one of claims 26 to 29 wherein the composition further comprises omega-6 fatty acids.

31. The method as set forth in claim 30 wherein the weight ration of omega-3 fatty acids to omega-6 fatty acids in the composition is from 1:2 to 1:4.

## Patentansprüche

1. Brust-Pad (Brusteinlage) zum Absorbieren von aus der Brust einer Frau austretender Flüssigkeit und zur Minimierung der Verunreinigung der Kleidung, welche die Frau trägt, wobei das Brust-Pad eine Vorderseite, die der Brust gegenüberliegt, und eine Rückseite, die der Kleidung gegenüberliegt, aufweist, wobei die Vorderseite 0,1 bis 30 g/m² einer Zusammensetzung zur Verbesserung der Gesundheit der Haut der Brust und der Brustwarze umfasst, die Omega-3-Fettsäuren enthält.

2. Brust-Pad nach Anspruch 1, in dem die Zusammensetzung außerdem essentielle Fettsäuren umfasst.

3. Brust-Pad nach Anspruch 1 oder 2, in dem die Zusammensetzung außerdem Omega-6-Fettsäuren umfasst.

4. Brust-Pad nach einem der Ansprüche 1 bis 3, in dem die Zusammensetzung 0,05 bis 1,5 Gew.-% Omega-3-Fettsäuren, bezogen auf das Gesamtgewicht der Zusammensetzung, umfasst.

5. Brust-Pad nach einem der Ansprüche 1 bis 3, in dem die Zusammensetzung 0,1 bis 1 Gew.-% Omega-3-Fettsäuren, bezogen auf das Gesamtgewicht der Zusammensetzung, umfasst.

6. Brust-Pad nach einem der Ansprüche 1 bis 5, bei dem ein Öl, ausgewählt aus der Gruppe, die besteht aus Fischöl, Olivenöl, Canolaöl, Walnussöl und Leinsamenöl, in die Zusammensetzung eingeführt worden ist, wobei das Öl Omega-3-Fettsäuren umfasst.

7. Brust-Pad nach einem der Ansprüche 1 bis 6, das außerdem ein Additiv, ausgewählt aus der Gruppe, die besteht aus einem natürlichen Anfeuchtungsfaktor, einem Feuchthaltemittel, Vitamin C, Vitamin E, Aloe und einem essbaren pflanzlichen Wirkstoff, umfasst.

8. Brust-Pad nach einem der Ansprüche 1 bis 7, in dem die Zusammensetzung einen pH-Wert von 3 bis 8 hat.

9. Brust-Pad nach einem der Ansprüche 1 bis 8, in dem die Zusammensetzung außerdem 40 bis 60 Gew.-% eines Verfestigungsmittels (Erstarrungsmittels), bezogen auf das Gesamtgewicht der Zusammensetzung, umfasst.

10. Brust-Pad nach einem der Ansprüche 1 bis 9, in dem die Zusammensetzung außerdem 1 bis 40 Gew.-% eines Fettalkohols, bezogen auf das Gesamtgewicht der Zusammensetzung, umfasst.

11. Brust-Pad nach einem der Ansprüche 1 bis 10, in dem die Zusammensetzung außerdem 0,1 bis 10 Gew.-%, eines Additivs, ausgewählt aus der Gruppe, die besteht aus Sterinen, Sterin-Derivaten und Mischungen davon, bezogen auf das Gesamtgewicht der Zusammensetzung, umfasst.

12. Brust-Pad nach einem der Ansprüche 1 bis 11, in dem die Zusammensetzung außerdem 0,1 bis 10 Gew.-% eines extrahierten pflanzlichen Wirkstoffs, bezogen auf das Gesamtgewicht der Zusammensetzung, umfasst.

13. Brust-Pad nach einem der Ansprüche 1 bis 12, in dem die Zusammensetzung außerdem 0,1 bis 10 Gew.-% eines weich machenden Agens, bezogen auf das Gesamtgewicht der Zusammensetzung, umfasst.

14. Brust-Pad nach einem der Ansprüche 1 bis 13, in dem die Zusammensetzung außerdem 1 bis 20 Gew.-% eines Viskositätsverbesserungsmittels, bezogen auf das Gesamtgewicht der Zusammensetzung, umfasst.

15. Brust-Pad nach einem der Ansprüche 1 bis 14, in dem die Zusammensetzung außerdem 0,5 bis 20 Gew.-% eines die rheologischen Eigenschaften modifizierenden Agens, bezogen auf das Gesamtgewicht der Zusammensetzung, umfasst.

16. Brust-Pad nach einem der Ansprüche 1 bis 14, in dem die Zusammensetzung außerdem 0,5 bis 10 Gew.-% eines die rheologischen Eigenschaften modifizierenden Agens, bezogen auf das Gesamtgewicht der Zusammensetzung, umfasst.

17. Brust-Pad nach Anspruch 6, in dem das Öl Leinsamenöl ist.

18. Brust-Pad nach Anspruch 3, bei dem ein Öl, ausgewählt aus der Gruppe, die besteht aus Maisöl, Baumwollsamenöl, Saffloröl und Sonnenblumenöl, in die Zusammensetzung eingeführt worden ist, wobei das Öl Omega-6-Fettsäuren umfasst.

19. Brust-Pad nach Anspruch 3, bei dem das Gewichtsverhältnis von Omega-3-Fettsäuren zu Omega-6-Fettsäuren in der Zusammensetzung 1 : 2 bis 1 : 4 beträgt.

20. Brust-Pad nach Anspruch 2, in dem die Zusammensetzung 0,05 bis 1,5 Gew.-% essentielle Fettsäuren, bezogen auf das Gesamtgewicht der Zusammensetzung, umfasst.

21. Brust-Pad nach Anspruch 1, in dem die Zusammensetzung 1 bis 15 Gew.-% Leinsamenöl, bezogen auf das Gesamtgewicht der Zusammensetzung, umfasst.

22. Brust-Pad nach Anspruch 21, in dem die Zusammensetzung 1 bis 10 Gew.-% Leinsamenöl, bezogen auf das Gesamtgewicht der Zusammensetzung, umfasst.

23. Brust-Pad nach Anspruch 21 oder 22, das außerdem 1 bis 15 Gew.-% essentielle Fettsäuren, bezogen auf das Gesamtgewicht der Zusammensetzung, umfasst.

24. Brust-Pad nach Anspruch 3, in dem die Zusammensetzung Linolsäure, α-Linolsäure, Eicosapentensäure und Docosahexensäure umfasst.

25. Brust-Pad nach Anspruch 24, in dem das Gewichtsverhältnis von Omega-3-Fettsäuren zu Omega-6-Fettsäuren in der Zusammensetzung 1 : 2 bis 1 : 4 beträgt.

26. Verfahren zur Ergänzung der Nahrungsmittelaufnahme eines gestillten Säuglings, wobei das Verfahren umfasst:
das Einführen einer Zusammensetzung auf eine Vorderseite eines Brust-Pads (einer Brusteinlage), das (die) von der den Säugling stillenden Frau getragen werden soll, wobei das Brust-Pad eine Vorderseite, die der Trägerin gegenüberliegt, und eine Rückseite, die der Kleidung gegenüberliegt, aufweist, wobei die Zusammensetzung Omega-3-Fettsäuren umfasst;
das Übertragen der Zusammensetzung von der Vorderseite des Brust-Pads auf die Brustwarze und die Brust der Frau während des Tragens, sodass die Omega-3-Fettsäuren mit der Brustwarze und der Brust der Frau in Kontakt kommen; und
das Übertragen der Omega-3-Fettsäuren von der Brustwarze und der Brust der Frau auf den Säugling während des Stillens des Säuglings, sodass die Omega-3-Fettsäuren von dem Säugling aufgenommen werden.

27. Verfahren nach Anspruch 26, bei dem das Brust-Pad 0,05 bis 1,5 Gew.-% Omega-3-Fettsäuren, bezogen auf das Gesamtgewicht der Zusammensetzung, umfasst.

28. Verfahren nach Anspruch 26 oder 27, bei dem ein Öl, ausgewählt aus der Gruppe, die besteht aus Fischöl, Olivenöl, Canolaöl, Walnussöl und Leinsamenöl, in die Zusammensetzung eingeführt wird, wobei das Öl Omega-3-Fettsäuren umfasst.

29. Verfahren nach einem der Ansprüche 26 bis 28, bei dem die Zusammensetzung außerdem essentielle Fettsäuren umfasst.

30. Verfahren nach einem der Ansprüche 26 bis 29, bei dem die Zusammensetzung außerdem Omega-6-Fettsäuren umfasst.

31. Verfahren nach Anspruch 30, bei dem das Gewichtsverhältnis von Omega-3-Fettsäuren zu Omega-6-Fettsäuren in der Zusammensetzung 1 : 2 bis 1 : 4 beträgt.

## Revendications

1. Coussinet d'allaitement pour l'absorption de fluides suintant du sein d'une femme et la réduction à un minimum de la souillure des vêtements portés par la femme, ledit coussinet d'allaitement ayant une face avant tournée vers le sein et une face arrière tournée vers le vêtement, ladite face avant comprenant de 0,1 g/m² à 30 g/m² d'une composition d'amélioration de la santé cutanée du sein et du mamelon, ladite composition comprenant des acides gras oméga-3.

2. Coussinet d'allaitement selon la revendication 1, dans lequel ladite composition comprend, en outre, des acides gras essentiels.

3. Coussinet d'allaitement selon la revendication 1 ou la revendication 2, dans lequel ladite composition comprend, en outre, des acides gras oméga-6.

4. Coussinet d'allaitement selon l'une quelconque des revendications 1 à 3, dans lequel la composition comprend de 0,05 % (par rapport au poids total de la composition) à 1,5 % (par rapport au poids total de la composition) d'acides gras oméga-3.

5. Coussinet d'allaitement selon l'une quelconque des revendications 1 à 3, dans lequel la composition comprend de 0,1 % (par rapport au poids total de la composition) à 1 % (par rapport au poids total de la composition) d'acides gras oméga-3.

6. Coussinet d'allaitement selon l'une quelconque des revendications 1 à 5, dans lequel une huile sélectionnée dans le groupe consistant en l'huile de poisson, l'huile d'olive, l'huile de canola, l'huile de noix et l'huile de lin est introduite dans la composition, ladite huile comprenant des acides gras oméga-3.

7. Coussinet d'allaitement selon l'une quelconque des revendications 1 à 6, comprenant en outre un additif sélectionné dans le groupe consistant en un facteur d'hydratation naturel, un humectant, la vitamine C, la vitamine E, l'aloès et une plante comestible.

8. Coussinet d'allaitement selon l'une quelconque des revendications 1 à 7, dans lequel la composition a un pH compris entre 3 et 8.

9. Coussinet d'allaitement selon l'une quelconque des revendications 1 à 8, dans lequel la composition comprend, en outre, de 40 % (par rapport au poids total de la composition) à 60 % (par rapport au poids total de la composition) d'un agent solidifiant.

10. Coussinet d'allaitement selon l'une quelconque des revendications 1 à 9, dans lequel la composition comprend, en outre, de 1 % (par rapport au poids total de la composition) à 40 % (par rapport au poids total de la composition) d'un alcool gras.

11. Coussinet d'allaitement selon l'une quelconque des revendications 1 à 10, dans lequel la composition comprend, en outre, de 0,1 % (par rapport au poids total de la composition) à 10 % (par rapport au poids total de la composition) d'un additif sélectionné dans le groupe consistant en les stérols, les dérivés de stérols, et leurs mélanges.

12. Coussinet d'allaitement selon l'une quelconque des revendications 1 à 11, dans lequel la composition comprend, en outre, de 0,1 % (par rapport au poids total de la composition) à 10% (par rapport au poids total de la composition) d'un extrait botanique.

13. Coussinet d'allaitement selon l'une quelconque des revendications 1 à 12 dans lequel la composition comprend, en outre, de 0,1 % (par rapport au poids total de la composition) à 10 % (par rapport au poids total de la composition) d'un émollient.

14. Coussinet d'allaitement selon l'une quelconque des revendications 1. à 13, dans lequel la composition comprend, en outre, de 1 % (par rapport au poids total de la composition) à 20 % (par rapport au poids total de la composition) d'un réhausseur de viscosité.

15. Coussinet d'allaitement selon l'une quelconque des revendications 1 à 14, dans lequel la composition comprend, en outre, de 0,5 % (par rapport au poids total de la composition) à 20 % (par rapport au poids total de la composition) d'un modificateur de rhéologie.

16. Coussinet d'allaitement selon l'une quelconque des revendications 1 à 14, dans lequel la composition comprend, en outre, de 0,5 % (par rapport au poids total de la composition) à 10 % (par rapport au poids total de la composition) d'un modificateur de rhéologie.

17. Coussinet d'allaitement selon la revendication 6, dans lequel l'huile est de l'huile de lin.

18. Coussinet d'allaitement selon la revendication 3, dans lequel une huile sélectionnée dans le groupe consistant en l'huile de maïs, l'huile de coton, l'huile de carthame et l'huile de tournesol est introduite dans la composition, ladite huile comprenant des acides gras oméga-6.

19. Coussinet d'allaitement selon la revendication 3, dans lequel le rapport pondéral entre les acides gras oméga-3 et les acides gras oméga-6 dans la composition va de 1:2 à 1:4.

20. Coussinet d'allaitement selon la revendication 2, dans lequel la composition comprend de 0,05 % (par rapport au poids total de la composition) à 1,5 % (par rapport au poids total de la composition) d'acides gras essentiels.

21. Coussinet d'allaitement selon la revendication 1, dans lequel ladite composition comprend de 1 % (par rapport au poids total de la composition) à 15 % (par rapport au poids total de la composition) d'huile de lin.

22. Coussinet d'allaitement selon la revendication 21, dans lequel la composition comprend de 1 % (par rapport au poids total de la composition) à 10 % (par rapport au poids total de la composition) d'huile de lin.

23. Coussinet d'allaitement selon la revendication 21 ou la revendication 22, comprenant en outre de 1 % (par rapport au poids total de la composition) à 15 % (par rapport au poids total de la composition) d'acide gras essentiels.

24. Coussinet d'allaitement selon la revendication 3, dans lequel la composition comprend de l'acide linoléique, de l'acide alpha linoléique, de l'acide éicosapenténoïque et de l'acide docosahexénoïque.

25. Coussinet d'allaitement selon la revendication 24, dans lequel le rapport pondéral entre les acides gras oméga-3 et les acides gras oméga-6, dans la composition, va de 1:2 à 1:4.

26. Procédé de supplémentation à la prise de nutriments d'un nourrisson nourri au sein, le procédé comprenant :
l'introduction d'une composition sur une face avant d'un coussinet d'allaitement devant être porté par la femme allaitant le nourrisson, ledit coussinet d'allaitement ayant une face avant tournée vers l'utilisatrice et une face arrière tournée vers le vêtement, ladite composition comprenant des acides gras oméga-3,
le transfert de la composition depuis la face avant du coussinet d'allaitement au mamelon et au sein de la femme au cours du port, de telle sorte que les acides gras oméga-3 viennent en contact avec le mamelon et le sein de la femme ; et
le transfert des acides gras oméga-3 depuis le mamelon et le sein de la femme au nourrisson, au cours de l'allaitement du nourrisson, de telle sorte que les acides gras oméga-3 sont ingérés par le nourrisson.

27. Procédé selon la revendication 26, dans lequel le coussinet d'allaitement comprend de 0,05 % (par rapport au poids total de la composition) à 1,5 % (par rapport au poids total de la composition) d'acides gras oméga-3.

28. Procédé selon la revendication 26 ou la revendication 27, dans lequel une huile sélectionnée dans le groupe consistant en l'huile de poisson, l'huile d'olive, l'huile de canola, l'huile de noix et l'huile de lin est introduite dans la composition, ladite huile comprenant des acides gras oméga-3.

29. Procédé selon l'une quelconque des revendications 26 à 28, dans lequel la composition comprend, en outre, des acides gras essentiels.

30. Procédé selon l'une quelconque des revendications 26 à 29, dans lequel la composition comprend, en outre, des acides gras oméga-6.

31. Procédé selon la revendication 30, dans lequel le rapport pondéral entre les acides gras oméga-3 et les acides gras oméga-6, dans la composition, va de 1:2 à 1:4.
